# EUROPEAN PATENT APPLICATION

(11) **EP 4 131 154 A1**
(43) Date of publication of application: **08.02.2023**
(21) Application number: 21189888.7
(22) Date of filing: 05.08.2021
(51) Int. Cl.: G06T 7/00, G06T 7/11

(54) **CORONARY ARTERY NARROWING DETECTION BASED ON PATIENT IMAGING AND 3D DEEP LEARNING**

(71) Applicant: Robovision, 9052 Zwijnaarde (BE)
(72) Inventor: HOLVOET, Thomas, Zwijnaarde (BE); WILLAERT, Stephane, Zwijnaarde (BE); WAEGEMAN, Tim, Zwijnaarde (BE)
(74) Representative: Rogiest, Wouter

(57) **Abstract**

The invention relates, amongst others, to a method for determining an FFR-related parameter value, comprising: providing a CT image comprising coronary arteries obtained from coronary CT angiography, CCTA; extracting, from said CT image and for each of said coronary arteries, a respective centerline; extracting, from said CT image and for each of said coronary arteries, a respective artery contour; and determining, based at least on a coronary artery model comprising said respective centerlines and said respective artery contours, said FFR-related parameter value; wherein said CT image is a 3D CT image comprising voxels, each voxel being associated with a radiodensity value, preferably a Hounsfield unit value; wherein said extracting of said respective centerlines comprises applying, on said 3D CT image comprising voxels, a first NN being a 3D NN trained with respect to the centerline; wherein said extracting of said respective artery contours comprises applying, on said CT image, a second NN trained with respect to a radius from the centerline; and wherein said determining of said FFR-related parameter value comprises applying, on said coronary artery model, a third NN trained with respect to FFR-related training data.

## Description

### Field of the invention

The present invention relates to medical imaging and related methods. More specifically, embodiments of the present disclosure relate to non-invasive coronary artery narrowing detection based on CT scans and 3D deep learning.

### Background art

There is a need for functional assessment of arterial capacity in order to screen a patient's health. Such screening is preferably based on hemodynamic characteristics, such as Fractional Flow Reserve (FFR), which are key indicators to diagnose a patient and, by extension, to determine the optimal treatment for a patient with arterial disease. Prior art assessments of these hemodynamic characteristics are based on invasive catheterizations. These allow to directly measure blood flow characteristics, such as pressure and flow velocity. However, invasive measurement techniques entail risks to the patient and furthermore constitute a significant cost to the healthcare system.

Noninvasive tests overcome these problems and have been developed based on patient imaging, particularly, computed tomography (CT). This allows to determine a patient-specific coronary artery model. This model may then be used to computationally simulate the blood flow and obtain FFR-related parameter values using computational fluid dynamics (CFD) with appropriate physiological boundary conditions and parameters. Examples of inputs to these patient-specific boundary conditions include the patient's blood pressure, blood viscosity and the expected demand of blood from the supplied tissue. These inputs may be derived from scaling laws and a mass estimation of the supplied tissue from the patient imaging. Although CFD-based estimations of blood flow characteristics have demonstrated a level of fidelity comparable to direct (invasive) measurements of the same quantity of interest, physical simulations demand a substantial computational burden that can make these virtual, noninvasive tests difficult to execute in a real-time clinical environment. On the other hand, prior art methods for obtaining a coronary artery model are overly complex and/or lack accuracy. Consequently, the present disclosure describes new methods for obtaining highly automated, fast and noninvasive estimations of blood flow characteristics that are computationally inexpensive.

(Zhi-Qiang WANG, Yu-Jie ZHOU, Ying-Xin ZHAO, Dong-Mei SHI, Yu-Yang LIU, Wei LIU, Xiao-Li LIU, Yue-Ping LI. Diagnostic accuracy of a deep learning approach to calculate FFR from coronary CT angiography. J Geriatr Cardiol 2019; 16(1): 42-48. doi: 10.11909/j.issn.1671-5411.2019.01.010), (Sironi A, Turetken E, Lepetit V, Fua P. Multiscale Centerline Detection. IEEE Trans Pattern Anal Mach Intell. 2016 Jul;38(7):1327-41. doi: 10.1109/TPAMI.2015.2462363), (Wolterink JM, van Hamersvelt RW, Viergever MA, Leiner T, Išgum I. Coronary artery centerline extraction in cardiac CT angiography using a CNN-based orientation classifier. Med Image Anal. 2019 Jan;51:46-60. doi: 10.1016/j.media.2018.10.005), WO 2018/057529 A1, WO 2018/015414 A1, US 9,668,699 B2, and US 2020/0205745 A1disclose related methods and systems, but do not enable fully automatized dertermination of FFR-related parameter values, and/or are overly complex.

EP3480730A1 discloses computer-implemented method for identifying features in 3D image volumes includes dividing a 3D volume into a plurality of 2D slices and applying a pre-trained 2D multi-channel global convolutional network (MC-GCN) to the plurality of 2D slices until convergence. However, EP3480730A1 does not disclose applying a 3D NN to the 3D image.

The present invention aims at addressing the issues listed above.

### Summary of the invention

According to a first aspect of the present invention, a method is provided for determining an FFR-related parameter value, preferably an FFR, comprising:
providing a CT image comprising coronary arteries obtained from coronary CT angiography, CCTA;
extracting, from said CT image and for each of said coronary arteries, a respective centerline;
extracting, from said CT image and for each of said coronary arteries, a respective artery contour; and
preferably, determining, based at least on a coronary artery model comprising said respective centerlines and said respective artery contours, said FFR-related parameter value;
wherein said CT image is a 3D CT image comprising voxels, each voxel being associated with a radiodensity value, preferably a Hounsfield unit value;
wherein said extracting of said respective centerlines comprises applying, on said 3D CT image comprising voxels, a first NN being a 3D NN trained with respect to the centerline;
wherein said extracting of said respective artery contours comprises applying, on said CT image, a second NN trained with respect to a radius from the centerline; and
wherein preferably said determining of said FFR-related parameter value comprises applying, on said coronary artery model, a third NN trained with respect to FFR-related training data.

A main advantage of such a method is the convenient automation and non-intrusive nature of the determination of an FFR-related parameter value.

In preferred embodiments, the method includes the step of determining, based at least on a coronary artery model comprising said respective centerlines and said respective artery contours, said FFR-related parameter value, wherein said determining of said FFR-related parameter value preferably comprises applying, on said coronary artery model, a third NN trained with respect to FFR-related training data. In other embodiments, the method does not include said step of determining said FFR-related parameter value, and instead is a method for determining a coronary artery model comprising said respective centerlines and said respective artery contours. In such other embodiments, the obtained coronary artery model may be used, e.g., for determining said FFR-related parameter value based on CFD and/or based on a 3D printing of said obtained coronary artery model.

(Sironi A, Turetken E, Lepetit V, Fua P. Multiscale Centerline Detection. IEEE Trans Pattern Anal Mach Intell. 2016 Jul;38(7):1327-41. doi: 10.1109/TPAMI.2015.2462363) discloses the handling of 2D and 3D image stacks but merely discloses a regression formulation applied on 2D images comprised in a 3D image stack. Moreover, the regression method is GradientBoost, see Sect. 3.1, which is one of the traditional regression methods known to the skilled person.

On the other hand, (Wolterink JM, van Hamersvelt RW, Viergever MA, Leiner T, Išgum I. Coronary artery centerline extraction in cardiac CT angiography using a CNN-based orientation classifier. Med Image Anal. 2019 Jan;51:46-60. doi: 10.1016/j.media.2018.10.005) discloses 2D centerline extraction and contour extraction but not the determination of FFR-related parameter values.

In preferred embodiments, said applying of said first NN for extracting said respective centerlines comprises generating a 3D heat map comprising a confidence value per voxel followed by performing a regression on said confidence values. This is in contrast with the approach of (doi: 10.1016/j.media.2018.10.005) which is based on semantic segmentation, which, as the inventors have found, is suboptimal particularly for centerline extraction. The semantic segmentation in (doi: 10.1016/j.media.2018.10.005) is carried out by means of CNNs and deep learning but does not consider regression. The problem hereby may be that centerline extraction based on segmentation leads to a binary approach with respect to pixels (in case of 2D images) either belonging to the centerline or not belonging to the centerline. Likewise, 3D approaches with voxels based on semantic segmentation without regression may attribute voxels only to mutually exclusive categories. Such an attribution of pixels or voxels, respectively, to mutually exclusive categories, leads to drastic loss in accuracy. Addressing this problem, the inventors surprisingly have found that a 3D NN, preferably a 3D CNN, if used as regressor, may outperform the methods for centerline and contour extraction known in the prior art. CNN architectures such as 3D U-net, while being commonly used for segmentation tasks, have proven particularly advantageous. This may relate to the ability of U-net to use only the valid part of each convolution without necessitating fully connected layers, which may make it more suitable for regression tasks than fully connected NNs and CNNs.

According to a second aspect of the present invention, a device is provided. The device comprises a processor and memory comprising instructions which, when executed by the processor, cause the device to execute a method according to the present invention.

According to a further aspect of the present invention, a computer program product is provided. The computer program product comprises instructions which, when carried out on a processor, cause the processor to carry out the steps of the method according to

Preferred embodiments and their advantages are provided in the description and the dependent claims.

### Brief description of the drawings

The present invention will be discussed in more detail below, with reference to the attached drawings.
Fig. 1 illustrates example embodiments of methods according to the invention.
Fig. 2A provides an example of FFR values of an artery relating to the invention.
Fig. 2B provides an example of a distribution of FFR values relating to the invention.

### Description of embodiments

The following descriptions depict only example embodiments and are not considered limiting in scope. Any reference herein to the disclosure is not intended to restrict or limit the disclosure to exact features of any one or more of the exemplary embodiments disclosed in the present specification.

Furthermore, the terms first, second, third and the like in the description and in the claims are used for distinguishing between similar elements and not necessarily for describing a sequential or chronological order. The terms are interchangeable under appropriate circumstances and the embodiments of the invention can operate in other sequences than described or illustrated herein.

Furthermore, the various embodiments, although referred to as "preferred" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising A and B" should not be limited to devices consisting only of components A and B, rather with respect to the present invention, the only enumerated components of the device are A and B, and further the claim should be interpreted as including equivalents of those components.

In this document, the term " FFR-related parameter", or, equivalently, "blood flow characteristic", is an umbrella term for any parameter characteristic of blood flow in coronary arteries of a patient. This may comprise, e.g., any or any combination of fractional flow reserve (FFR), instantaneous wave-free ratio (iFR), pressure, flow, pressure gradient, flow rate, proximal pressure, distal pressure, medial pressure, microvascular resistance (MVR), stenosis resistance (SR), coronary flow reserve (CFR), absolute coronary flow, Doppler flow, as known to the skilled person, and disclosed, e.g., in (Morris, P D, Gosling, R, et al. A novel method for measuring absolute coronary blood flow and microvascular resistance in patients with ischaemic heart disease. Cardiovascular Research, 2020, cvaa220, doi:10.1093/cvr/cvaa220). The term "FFR" is thereby used with reference to both a technique and a parameter. Particularly, the technique of FFR may relate to, e.g., coronary catheterization to measure pressure differences across a coronary artery stenosis caused by, e.g., atherosclerosis, typically with the aim of determining the likelihood that the stenosis impedes oxygen delivery to the heart muscle (myocardial ischemia). Related, the FFR parameter, or FFR, is preferably defined as the pressure distal to a stenosis relative to the pressure proximal to the stenosis, yielding a fraction being an absolute number. Equivalently or alternatively, the FFR expresses the maximal flow down a vessel in the presence of a stenosis compared to the maximal flow in the hypothetical absence of the stenosis. Equivalently or alternatively, the FFR may express any fraction of a second pressure and a first pressure at respective second and first positions within a coronary artery, wherein the first value is proximal (or more proximal) and the second value is distal (or more distal). Related, an "FFR-related parameter" may refer to any quantitative parameter characteristic of a blood flow within a coronary artery, and may relate to any absolute measurement (flow, pressure, resistance) with respect to a single point in the artery, and/or any relative fraction of a first measurement and a second measurement, either with respect to a single point in the artery (e.g., with first and second measurement relating to different time instants) or with respect to a first and second point in the artery. Related, "FFR-related training data" may refer to any data available for either said FFR-related parameter or another FFR-related parameter. In other words, the FFR-related training data may relate to a second FFR-related parameter which may or may not be different from the FFR-related parameter for which the FFR-related parameter value is being determined. Therein, it is assumed that the data for the second FFR-related parameter has sufficient relevance for the determination of the FFR-related parameter value. In embodiments, the training data may be obtained from any source. The training data may relate to any invasive or non-invasive measurement or calculation as described in this document.

The term neural network, NN, refers to any neural network model. The NN may comprise any or any combination of a multilayer perceptron, MLP, a convolutional neural network, CNN, and a recurrent neural network, RNN. A trained NN relates to training data associated with a neural network based model.

Neural networks need to be trained to learn the features that optimally represent the data. Such deep learning algorithms includes a multilayer, deep neural network that transforms input data (e.g. images, CT images) to outputs while learning higher level features. Successful neural network models for image analysis are referred to semantic segmentation NNs, and find application in a variety of tasks, which is not limited to semantic segmentation alone, but may also include, e.g., regression. One example is the so-called convolutional neural network (CNN). CNNs contain many layers that transform their input using kernels, also known as convolution filters, consisting of a relatively small sized matrix. Other successful neural network models for image analysis are instance segmentation NNs. As known to the skilled person, instance segmentation NNs differ from semantic segmentation NNs in terms of algorithm and output, even in cases where the input, e.g. the images, are identical or very similar.

In general, semantic segmentation may relate, without being limited thereto, to detecting, for every pixel (in 2D) or voxel (in 3D), to which class the pixel/voxel belong. Regression may relate to determining other quantitative or qualitative metadata in relation to the pixel/voxel.

Instance segmentation, on the other hand, may relate, without being limited thereto, to detecting, for every pixel/voxel, a belonging instance with respect to a plurality of similar objects. It may detect each distinct object of interest in an image.

In embodiments, 2D instance segmentation, preferably operating on 2D images, relates to Mask R-CNN, DeepMask, and/or TensorMask.

In embodiments, 3D instance segmentation, preferably operating on a 3D point cloud generated from 2D images, relates to 3D-BoNet and/or ASIS.

In embodiments, the at least one trained NN is rotation equivariant. In embodiments, the NN is translation and rotation equivariant.

In embodiments, said first, second and/or third NN may comprise a semantic segmentation NN used for regression and/or segmentation. In embodiments, said first, second and/or third NN may comprise a NN that is commonly referred to as a semantic segmentation NN, but is used instead for regression. U-net is found to be particularly suitable due to increased speed and/or increased reliability, enabled by data augmentation and elastic deformation, as described for the 2D case in more detail in, e.g., (Ronneberger, Olaf; Fischer, Philipp; Brox, Thomas (2015). "U-net: Convolutional Networks for Biomedical Image Segmentation. arXiv:1505.04597").

In embodiments, the first, second and/or third NN may relate to a rotation equivariant NN. are known for specific applications, see, e.g., the "e2cnn" software library, see (Maurice Weiler, Gabriele Cesa, General E(2)-Equivariant Steerable CNNs, Conference on Neural Information Processing Systems (NeurIPS), 2019). The inventors have found such rotation equivariant NNs to be particularly useful for objects with symmetry along an axis such as arteries.

In embodiments, the first, second and/or third NN may comprise any or any combination of a graph NN, a recurrent NN (RNN), a bidirectional neural network (BRNN or BiRNN), a U-net, a Deeplabv3+.

The acquisition of CT images may be conducted according to methods known to the skilled person, and may involve, e.g., contrast agent and ECG gating to acquire images at diastolic phase when contrast agent is fully perfused in the coronary arteries. The acquisition of CT images may involve adequate techniques for imrproving acquisitation quality and/or preprocessing, such as ECG synchronization, motion blurring suppression, stair-step artifact compensation, and/or noise reduction.

The acquisition of FFR data, preferably FFR training data for the purpose of training at least one of said first, second and third NN, may be conducted invasively or non-invasively. It may be performed invasively according to methods known to the skilled person. This may relate, e.g., to combining an instrument and a coronary pressure wire. Measurement may for instance comprise a step of calibration and equalization, followed by advancing the pressure wire distally to a certain zone, e.g., a potential stenosis zone, and then further until a smooth coronary segment is reached. Pullback FFR data may, e.g., be recorded from the immediate downstream of a distal stenosis to the ostium. Pullback FFR data may relate to, e.g., the techniques disclosed in (Sonck, J, Collet, C, Mizukami, T, et al. Motorized fractional flow reserve pullback: Accuracy and reproducibility. Catheter Cardiovasc Interv. 2020; 96: E230- E237, doi:10.1002/ccd.28733). The acquisition of FFR data may also be performed non-invasively, e.g., by performing measurements, such as a single FFR measurement and/or a motorized FFR pullback measurement, on a 3D printed geometry of one of said coronary arteries. The 3D printed geometry may comprise geometries available from an external source and/or may comprise geometries printed based at least on a training coronary artery model determined in the same way as the coronary artery model determined according to the invention. In embodiments, the pullback FFR measurement generates a plurality of at least three measurement points, more preferably at least four or five or six or seven or eight or nine measurement points, most preferably at least ten measurement points, as this may correspond to a quasi-continuous or continuous measurement along the artery.

In embodiments, said applying of said first NN for extracting said respective centerlines comprises generating a 3D heat map comprising a confidence value per voxel followed by performing a regression on said confidence values. This may have the advantage of providing more accurate determination of the centerline. Additionally, or complementarily, this may have the advantage of enabling a more accurate artery contour extraction, since it enables considering the centerline not as a mere "binary" set of voxels, but as a more detailed confidence map for possible points belonging to the centerline.

In embodiments, said extracting of said respective artery contours comprises determining a seed based on a maximum confidence value on said 3D heat map corresponding to a voxel not belonging to said centerline. In embodiments, this relates to said extracting said respective centerlines comprising generating a 3D heat map comprising a confidence value per voxel followed by performing a regression on said confidence values. In other embodiments, the centerlines may be determined differently, and the 3D heat map is determined dedicatedly for artery contour extraction.

In embodiments, said first NN is a 3D U-Net or a 3D Deeplabv3+. The inventors have found that such NNs, if used as regressor, may outperform the methods for centerline and contour extraction known in the prior art.

In embodiments, said third NN is applied on the combination of said coronary artery model and voxel portions of said 3D CT image, wherein each voxel is associated with a radiointensity value. This may provide for more and more relevant input for the NN, enabling more accurate determination of the FFR-related parameter value. In embodiments, this relates to voxel portions that are non-overlapping. In embodiments this relates to at least one voxel portion at the centerline. In embodiments this relates to voxels portions at the artery contour. In embodiments this relates to non-overlapping voxel portions at the centerline and at the artery contour.

In embodiments, said voxel portions relate to respective voxel cuboids extracted from said 3D CT image at respective positions on the extracted artery contour, wherein more preferably said voxel cuboids have a cuboid size of x by y by z voxels whereby min(x,y,z) is three or more, preferably five or more, most preferably wherein said voxel cuboids are voxel cubes.

In embodiments, said third NN is a graph NN. The inventors have found that such NNs, which are associated with a graph structure, may be particularly adequate for problems involving artery contours, which have a specific, well-known geometry that may correspond to a graph abstraction.

In embodiments, said method comprises the further step of training of said third NN with respect to said FFR-related training data, said training data comprising at least one distribution of flow, pressure or resistance along one of said coronary arteries. In embodiments, said distribution of flow, pressure or resistance comprises at least one measured distribution of epicardial resistance measured in a patient. In embodiments, said at least one measured distribution of epicardial resistance in said patient relates to a measurement based on motorized FFR pullback. In embodiments, this relates to the motorized FFR pullback as further disclosed in, e.g., (Sonck, J, Collet, C, Mizukami, T, et al. Motorized fractional flow reserve pullback: Accuracy and reproducibility. Catheter Cardiovasc Interv. 2020; 96: E230- E237, doi:10.1002/ccd.28733). In embodiments, said at least one distribution of flow, pressure or resistance comprises at least one distribution calculated based on CFD, as further disclosed in, e.g., (Morris, P D, Gosling, R, et al. A novel method for measuring absolute coronary blood flow and microvascular resistance in patients with ischaemic heart disease. Cardiovascular Research, 2020, cvaa220, doi:10.1093/cvr/cvaa220). In embodiments, said at least one distribution of flow, pressure or resistance comprises at least one measured distribution measured on a 3D printed geometry of one of said coronary arteries, said geometry printed based at least on a training coronary artery model determined in the same way as said coronary artery model. This may relate, e.g., to methods involving 3D printing as disclosed in (Morris, P D, Gosling, R, et al. A novel method for measuring absolute coronary blood flow and microvascular resistance in patients with ischaemic heart disease. Cardiovascular Research, 2020, cvaa220, doi:10.1093/cvr/cvaa220).

In embodiments, said second NN is a graph NN. The inventors have found that such NNs, which are associated with a graph structure, may be particularly adequate for problems involving artery contours, which have a specific, well-known geometry that may correspond to a graph abstraction. In embodiments, both the second NN and the third NN are graph NNs.

In embodiments, said extracting of said respective artery contours comprises extracting both respective outer artery contours and respective inner artery contours. This has the advantage of capturing artery wall thickness, which may be highly relevant to the determination of FFR-related parameters.

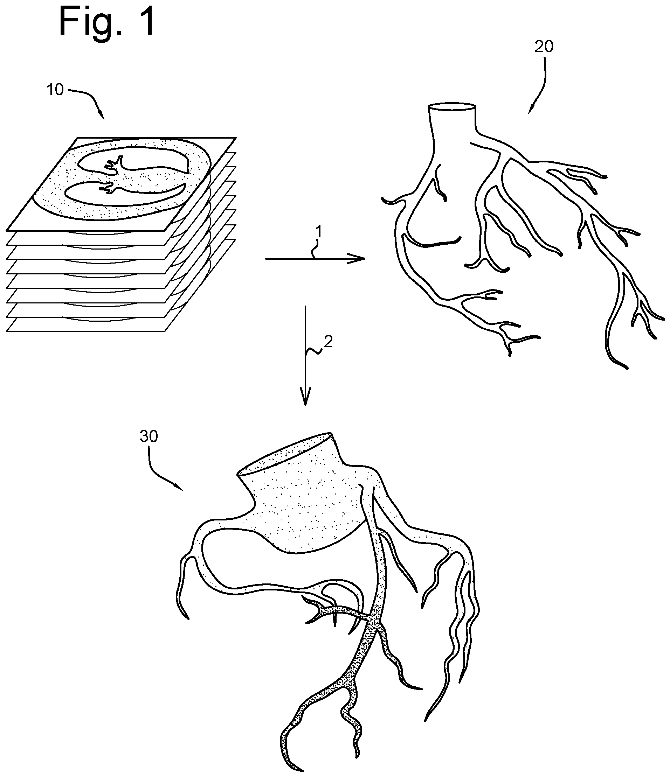

Example embodiments of the invention will be described with reference to Figs. 1-2B.

### Example 1: example methods according to the invention

Fig. 1 illustrates example embodiments of methods according to the invention. This relates to methods for determining an FFR-related parameter value, such as the FFR value of Example 2.

The example method comprising several steps. In a first step, a CT image (10) comprising coronary arteries obtained from coronary CT angiography, CCTA, is provided. The CT image (10) is a 3D CT image comprising voxels, each voxel being associated with a Hounsfield unit value. The CT image being a 3D image comprising voxels, it is equivalent to a stack of 2D images of pixels, as illustrated in Fig. 1.

In a first phase (1), the centerlines and contours are extracted. This comprises, first, the step of extracting, from said CT image and for each of said coronary arteries, a respective centerline. Second, this comprises the step of extracting, from said CT image and for each of said coronary arteries, a respective artery contour. The result of these steps, or, equivalently, of the first phase (1), is a coronary artery model (20).

In a second phase (2), the FFR-related parameter value is determined. This may, e.g., be the FFR, as in Example 2, but may be any FFR-related parameter value. This determination is based at least on the coronary artery model (20) comprising said respective centerlines and said respective artery contours.

By determining a sufficient number of FFR-related parameter values, a textured representation (40) of the artery contour model may be generated. Herein, the texture may provide a visual indication of the numerical value of the FFR-related parameter. For instance, in the case of FFR with value ranging between 0 and 1, higher FFR values may be associated with sparser texture and lower FFR values with denser texture, as illustrated. Additionally, or complimentarily, higher FFR values may be associated with a first texture color and lower FFR values with a second texture color, with a color gradient in-between (not illustrated).

The example method comprises the use of several NNs.

The extracting of said respective centerlines comprises applying, on said 3D CT image comprising voxels, a first NN being a 3D NN trained with respect to the centerline. Hereby, training may be performed with invasive training data and/or non-invasive training data. The first NN is a U-net trained on centerline data. It generates a 3D heat map comprising a confidence value per voxel. This allows to perform a regression on said confidence values, e.g., by means of a gradient-based technique. Furthermore, the 3D heat map enables improved artery contour extraction (see below). As for the centerline extraction, based on the regression, and preferably some criterion on continuity of different portions of the centerline, the centerlines are determined.

The extracting of said respective artery contours comprises applying, on said CT image, a second NN trained with respect to a radius from the centerline. Again, training may be performed with invasive training data and/or non-invasive training data. For the extraction of the respective artery contours, multiple seeds are determined along the centerline, preferably based on a maximum confidence value on said 3D heat map. This may correspond to a voxel belonging to said centerline but may as well lie outside the centerline, since the quality of seeds is primarily determined by the confidence value, rather than being exactly on the centerline. In this regard, contour extraction according to Example 1 uses an augmented input, using not only the extracted (continuous) centerlines but also the heat map generated during centerline extraction, leading to a more accurate performance with respect to contour extraction. For the contour extraction, a graph NN is used which is trained with respect to a radius between the seed/centerline and the contour. Thereby, the graph structure of the NN, suitable captures the geometry of the coronary arteries. In embodiments, said extracting of said respective artery contours comprises extracting both respective outer artery contours and respective inner artery contours. This has the advantage of capturing artery wall thickness, which may be highly relevant to the determination of FFR-related parameters. In embodiments, the graph NN is applied, for respective positions on the centerline, to 2D slices extracted from the CT image at the respective position along a direction orthogonal to the centerline. In other embodiments, the graph NN is applied, for respective positions on the centerline, to 3D cuboids, i.e. voxel portions with cuboid shape and with each voxel associated with a radiointensity value, extracted from the CT image in the vicinity of the respective position.

The determining of said FFR-related parameter value comprises applying, on said coronary artery model, a third NN trained with respect to FFR-related training data. Also here, training may be performed with invasive training data and/or non-invasive training data.

In this example, the training data is a distribution, i.e. a pullback along the centerline, obtained from motorized FFR pullback, similar to, e.g., the data illustrated in Example 2. Thereby, the raw data obtained from motorized FFR pullback may be pre-processed based on CFD to detect outliers and improve continuity. In another example, the training data may be obtained from actual fluid dynamics testing on a 3D printed geometry of one of said coronary arteries, whereby said geometry that is printed is based on a training coronary artery model determined in the same way as the coronary artery model.

The third NN is applied on the combination of the coronary artery model and voxel portions of said 3D CT image. The voxel portions are non-overlapping voxel portions at the centerline and at the artery contour having a cuboid shape with size of x by y by z voxels whereby min(x,y,z) is three, five, ten, or more than ten. The third NN may be either a BiRNN or a graph NN.

### Example 2: example embodiments with 2D segmentation according to the invention

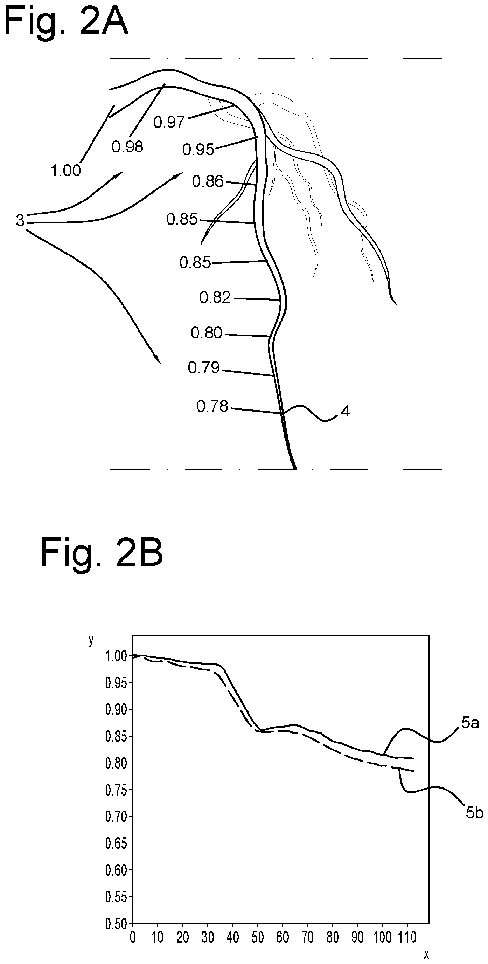

Fig. 2A provides an example of FFR values of an artery relating to the invention. Fig. 2B provides an example of a distribution of FFR values relating to the invention. Particularly, Fig. 2A illustrates an example artery contour model, wherein a distribution of FFR values (3) across the coronary artery is shown, ranging from 1.00 at the proximal end to 0.78 near the distal end (4).

In examples, Fig. 2A may relate to the output of the method according to the invention, wherein Fig. 2A illustrates both the artery contour model and the FFR-related parameter values, i.e. FFR values (3) generated by the method, as generated based on an input CT image and the trained NNs.

In other examples, Fig. 2A may relate to an intrusive measurement based on motorized FFR pullback, with FFR values (3) displayed as measured. The intrusive measurement may be used as training data for training the third NN. The position with FFR value equal to 1.00 may correspond to the proximal position of the pressure wire, whereas the position with FFR value equal to 0.78 may correspond to the distal position of the pressure wire. Fig. 2B may thereby show the FFR (y) (without unit) as function of distance (x), expressed in mm. The different curves (5a, 5b) may relate to respective first and second pullbacks.

In yet other examples, Fig. 2A may relate to a non-intrusive computation of FFR, based, e.g., on CFD. Such CFD-computed values may be used as training data for training the third NN.

In still other examples, Fig. 2A may relate to a non-intrusive determination of FFR, based, e.g., on fluid dynamics measurement on a 3D printed artery. Such 3D printed artery may be obtained from a coronary artery model determined by the centerline extraction and artery contour extraction according to the invention. Such values measured by fluid dynamics may be used as training data for training the third NN.

## Claims

1. A method for determining an FFR-related parameter value (3), comprising:
providing a CT image (10) comprising coronary arteries obtained from coronary CT angiography, CCTA;
extracting, from said CT image (10) and for each of said coronary arteries, a respective centerline;
extracting, from said CT image (10) and for each of said coronary arteries, a respective artery contour; and
determining, based at least on a coronary artery model (20) comprising said respective centerlines and said respective artery contours, said FFR-related parameter value (3);
wherein said CT image (10) is a 3D CT image comprising voxels, each voxel being associated with a radiodensity value, preferably a Hounsfield unit value;
wherein said extracting of said respective centerlines comprises applying, on said 3D CT image (10) comprising voxels, a first NN being a 3D NN trained with respect to the centerline;
wherein said extracting of said respective artery contours comprises applying, on said CT image, a second NN trained with respect to a radius from the centerline; and
wherein said determining of said FFR-related parameter value comprises applying, on said coronary artery model, a third NN trained with respect to FFR-related training data.

2. Method of claim 1, wherein said applying of said first NN for extracting said respective centerlines comprises generating a 3D heat map comprising a confidence value per voxel followed by performing a regression on said confidence values.

3. Method of claim 2, wherein said extracting of said respective artery contours comprises determining a seed based on a maximum confidence value on said 3D heat map corresponding to a voxel not belonging to said centerline.

4. Method of claims 2-3, wherein said first NN is a 3D U-Net or a 3D Deeplabv3+.

5. Method of claims 1-4, wherein said third NN is applied on the combination of said coronary artery model (20) and voxel portions of said 3D CT image; preferably wherein said voxel portions relate to respective voxel cuboids extracted from said 3D CT image at respective positions on the extracted artery contour, wherein more preferably said voxel cuboids have a cuboid size of x by y by z voxels whereby min(x,y,z) is three or more, preferably five or more, most preferably wherein said voxel cuboids are voxel cubes.

6. Method of claims 1-5, wherein said third NN is a graph NN.

7. Method of claims 1-6, wherein said method comprises the further step of training of said third NN with respect to the FFR-related training data, said training data comprising at least one distribution of flow, pressure or resistance at a plurality of positions along one of said coronary arteries.

8. Method of claim 7, wherein said at least one distribution of flow, pressure or resistance comprises at least one measured distribution of epicardial resistance.

9. Method of claim 8, wherein said at least one measured distribution of epicardial resistance relates to a motorized FFR pullback (5a, 5b).

10. Method of claims 7-9, wherein said at least one distribution of flow, pressure or resistance comprises at least one distribution calculated based on a CFD model.

11. Method of claims 7-10, wherein said at least one distribution of flow, pressure or resistance comprises at least one measured distribution measured on a 3D printed geometry of one of said coronary arteries, said geometry printed based at least on a training coronary artery model determined in the same way as said coronary artery model (20).

12. Method of claims 1-11, wherein said second NN is a graph NN.

13. Method of claims 1-12, wherein said extracting of said respective artery contours comprises extracting both respective outer artery contours and respective inner artery contours.

14. A device comprising a processor and memory comprising instructions which, when executed by the processor, cause the device to execute a method according to any of claims 1-13.

15. A computer program product comprising instructions which, when carried out on a processor, cause the processor to carry out the steps of the method according to claims 1-13.
